# EUROPÄISCHE PATENTSCHRIFT

(11) **EP 0 160 897 B1**
(45) Veröffentlichungstag und Bekanntmachung des Hinweises auf die Patenterteilung: **19.05.1993**
(21) Anmeldenummer: 85105083.1
(22) Anmeldetag: 26.04.1985
(51) Int. Cl.: C12P 21/08, C07K 15/00, G01N 33/574, A61K 47/00, A61K 39/395

(54) **Monoklonale Antikörper, Verfahren zu ihrer Herstellung sowie ihre Verwendung**
Monoclonal antibodies, process for preparing them and their use
Anticorps monoclonaux, leur procédé de préparation et leur application

(30) Priorität: 07.05.1984 DE 3416774
(43) Veröffentlichungstag der Anmeldung: 13.11.1985
(60) Teilanmeldung: 92113308.8
(73) Patentinhaber: BEHRINGWERKE Aktiengesellschaft, 35001 Marburg (DE)
(72) Erfinder: Bosslet, Klaus, Dr., D-3550 Marburg (DE); Sedlacek, Hans-Harald, Dr., D-3550 Marburg (DE)

(56) Entgegenhaltungen:
- EP-A- 0 141 079
- EP-A- 0 153 114
- EP-A- 0 160 446
- WO-A-81/01469
- CHEMICAL ABSTRACTS, Band 94, Heft 17, 27. April 1981, Seite 577, Zusammenfassung Nr. 137538s, Columbus, Ohio, US; K.F. MITCHELL: "A carcinoembryonic antigen (CEA) specific monoclonal hybridoma antibody that reacts only with high-molecular-weight CEA" & CANCER IMMUNOL. IMMUNOTHER. 1980, 10(1), 1-5
- PROCEEDINGS OF THE NATIONAL ACADEMY OF SCIENCES OF USA, Band 77, Heft 1, Januar 1980, Seiten 563-566; R.S. ACCOLLA et al.: "Monoclonal antibodies specific for carcinoembryonic antigen and produced by two hybrid cell lines"

## Beschreibung

Die Erfindung betrifft monoklonale Antikörper(MAK), die an definierte zytoplasmatische, membranassoziierte und sezernierte Antigene binden. Diese Antikörper können als Diagnostikum oder als Wirkstoff bzw. Wirkstoffträger verwendet werden.

Die Technik der Immunisierung mit definierten, isolierten Antigenen und der Produktion von Antikörpern gegen solche Antigene ist bekannt. Es ist auch bekannt, mit ungereinigtem Antigenmaterial zu immunisieren und diejenigen Antikörper zu selektieren, die eine bestimmte Komponente einer solchen Antigenmischung erkennen.

Bei dem Versuch, solche Antikörper zu induzieren, ist uns gelungen, monoklonale Antikörper zu selektieren, die unter nicht reduzierenden Bedingungen mit folgenden Proteinantigenen bzw. bestimmten Epitopen auf diesen Proteinantigenen reagieren.
- Antigen 2:: Ein Glykoproteinkomplex vom MW >200 KD unter nicht reduzierenden Bedingungen.

Antigen 2 trägt ein Epitop, das auf der Zellmembran der Oat-75 kleinzelligen Lungenkarzomzellinie sowie auf von der Oat-75 abgeleiteten und auf der nackten Maus transplantierten Tumoren dem MAK 278/105 vom IgG₃-Isoptyp zugänglich ist. Das Epitop ist in Gefäßen nachzuweisen, beispielsweise in Kryostatschnitten als auch auf formaldehydbehandelten paraffineingebetteten Materialien von Normal- und Tumorgewebe. Hier ist es bevorzugt in Endothelzellen anzutreffen, kann jedoch auch in extrazellulären Bereichen wie beispielsweise in Teilen der Basalmembran oder der Lamina elastica interna angetroffen werden. Es ist in Megakaryozyten nicht nachweisbar, dafür aber im Kapillarschlingenendothel der Glomeruli (im Gegensatz zu F VIIIR:AG). Das Epithel in der Bowmann schen Kapsel ist nicht reaktiv. Naturgemäß ist es besonders angereichert in Tumoren des Gefäßsystems. Das Epitop ist beispielsweise auch in humanen Nabelschurendothelzellen sowohl auf der Zellmembran (nach Glutaraldehydfixierung) wie auch im Zytoplasma (pünktchenförmige Komponente um den Kern und intrazytoplasmatische granuläre Verteilung) nachzuweisen.
Desweiteren exprimieren humane Darmbeinkammvenenendothelzellen das Epitop, wohingegen in Rinderlungenaortaendothelzellen das Epitop nicht nachweisbar ist.
Auf der Zellmembran menschlicher peripherer Blutleukozyten ist das Epitop nicht nachweisbar. Neben der oben angedeuteten Resistenz des Epitops im Gewebe gegen Formaldehydfixierung und Paraffineinbettung ist es weiterhin resistent gegen Neuraminidasebehandlung aber empfindlich gegen Perjodsäureoxidation.
Der MAK 278/105 scheint aufgrund seiner Eigenschaften zur Erkennung von vom Endothel abgeleiteten Tumoren sowie bei chronischen Entzündungen geeignet zu sein.

Eine Applikation ist der nichtinvasive Nachweis von Lymphknotenmetastasen beim Mammakarzinom durch Injektion des radioaktiv markierten MAK in die Interdigitalfalten, in die drainierenden Lymphbahnen oder intravenös. Zusätzliche Anwendungsgebiete sind die Immunszintigraphie und die Immuntherapie vor allem bei Plattenepithelkarzinomen der Lunge, aber auch anderen histologischen Lungenkarzinomtypen, dem duktalen Mammakarzinom sowie bestimmten exokrinen Pankreaskarzinomen.

Die für die Erzeugung obiger monoklonaler Antikörper benutzte Methodik ist in der Deutschen Offenlegungsschrift 33 29 184 (EP-A-141079) auf den Seiten 3, 4 und 5 beschrieben.

Gegenstand der Erfindung sind, Monoklonale Antikörper, die ein Epitop, das auf der Zellmembran der OAT-75 kleinzelligen Lungenkarzinomzellinie, auf von der OAT-75 abgeleiteten und auf der nackten Maus transplantierten Tumoren, in Gefäßen, im Kapillarschlingenendothel der Glomeruli, in Darmbeinkammvenenendothelzellen und das auf einem Glykoprotein-Antigen (AG2) mit einem Molekulargewicht > 200 kD unter nicht reduzierenden Bedingungen, das serologisch nicht mit FVIIIR:AG identisch ist, nachweisbar ist, erkennen und durch die durch Immunisieren von Säugetieren mit Zellen der Zellinie OAT-75 hergestellt werden.

Dies Antigen ist eindeutig durch das vorstehend angegebene Vorkommen auf bestimmten Zellen und Geweben charakterisiert sowie den Charakteristika der auf diese Antigen vorkommenden durch die jeweiligen MAK definierten Epitope.

Gegenstand der Erfindung ist weiterhin ein Verfahren zur Herstellung eines der oben beschriebenen monoklonalen Antikörper, dadurch gekennzeichnet, daß Säugetiere mit Zellen der Oat-75-Zellinie, immunisiert, Milzzellen aus einem solchen immunisierten Tier entnommen, mit der Zellinie X 63-Ag8653 fusioniert, die Hybridome selektioniert und der monoklonale Antikörper gewonnen werden.

Als Immunogen zur Induktion vom MAK 278/105 dient die Oat-75 Zellinie.

Säugetiere, vorzugsweise Mäuse, werden mit Zellen bzw. Antigenen einer oder mehrerer dieser Zellinien immunisiert und die Milzzellen aus solchen Tieren mit der X63 Ag8653 Zellinie fusioniert.

Es können auch zum Beispiel humane lymphoide Zellen von Tumorpatienten bzw. gesunden Menschen entnommen, in vitro immunisiert und mit einer menschlichen Myelomzelle fusioniert werden. Auch die Technik der EBV-Transformation bzw. die Transfektion mit DNA (onkogen transfer) kann benutzt werden, um die humanen lymphoiden Zellen zur permanenten Teilung anzuregen.

Die entstehenden Hybridome werden getestet, ob sie Antikörper der gewünschten Spezifität enthalten. Unter den ausgetesteten Hybridomüberständen befanden sich einige, die Antikörper der oben beschriebenen Spezifitäten enthielten. Die diese Antikörper sezernierenden Hybridome wurden kloniert und die von diesen Hybridomaklonen gewonnenen monoklonalen Antikörper benutzt, um das von ihnen erkannte Antigen immunchemisch zu charakterisieren. Das Molekulargewicht wird bestimmt im Vergleich zu kommerziell erhältlichen Markern. Die Antigene können affinitätschromatographisch gereinigt werden.

Zusätzlich wurde die Bindung der monoklonalen Antikörper an histologische Präparate mittels der indirekten Immunperoxidasetechnik gemessen (J.Clin.Pathol.(1979),32,971).

Weiterhin wurde die molekulare Charakterisierung des von den monoklonalen Antikörpern erkannten Antigens zusätzlich zur Westernblotanalyse mittels Radioimmunpräzipitation (Behring Institute Mitteilungen (1984), 74, 27-34) durchgeführt.

Die molekulare Charakterisierung der von den MAK erkannten Epitope auf humanem Gewebe erfolgte wie im folgenden beschrieben:
4-6 µm dicke Schnitte kryopräservierter humaner, die entsprechenden Epitope tragenden Gewebe wurden nach dem Antrocknen auf dem Objektträger (30-60 min bei Raumtemperatur) für 1-2 sec in Aceton fixiert. Anschließend wurden sie
a) für die Perjodatoxidation in PBS pH 7,2 mit 1 g/l BSA 10 min gewaschen, kurz in PBS pH 7,2 eingetaucht (gespült) und 1 Stunde bei Raumtemperatur in der feuchten Kammer mit 1 g/100 ml Perjodsäure in PBS pH 7,2 inkubiert. Anschließend wird 3 x 5 min in 1 g/l BSA in PBS pH 7,2 gewaschen und die Schnitte mit Ziege-Normalserum inkubiert, wie in J.Clin.Pathol. (1979) 32, 971 mit den zu testenden MAK weiterbehandelt und deren Bindung an die Gewebe mittels der indirekten Immunperoxidasetechnik nachgewiesen.
b) für die Neuraminidasebehandlung 2 x 10 min in 0,05 M Na⁺, CA⁺⁺ Acetatpuffer pH 5,5 gewaschen und 1 std mit verschiedenen Mengen von Vibrio cholerae Neuraminidase (100 U/ml, 10 U/ml, 1 U/ml, 0,1 U/ml) in obigem Acetatpuffer bei Raumtemperatur in der feuchten Kammer inkubiert. Nach zweimaligem Waschen in Acetatpuffer und zweimaligem Waschen in PBS pH 7,2 mit 1 g/l BSA werden die Schnitte mit Ziege-Normalserum inkubiert, wie in J.Clin.Pathol. (1979) 32, 971 mit den zu testenden MAK weiterbehandelt und deren Bindung an die Gewebe mittels der indirekten Immunperoxidasetechnik nachgewiesen.

Anstatt von 4-6 µm dicken kryopräservierten Gewebeschnitten werden zur Austestung der Formaldehyd- und Paraffinresistenz des von dem entsprechenden MAK erkannten Epitops, in Formaldehyd fixierte und paraffineingebettete Gewebe genommen, die in einem Mikrotom geschnitten, deparaffiniert (B. Romeis, Mikroskopische Techniken, 16. Auflage, S. 145, Kapitel 564, 1968) und wie in J.Clin.Pathol. (1979) 32, 971 beschrieben für die indirekte Immunperoxidasetechnik weiterbehandelt werden.

Aufgrund ihrer Reaktivität mit Antigen 1 können die charakterisierten monoklonalen Antikörper zur diagnostischen Erkennung und Unterscheidung der 72 KD-Antigen tragenden kleinzelligen Lungentumoren von anderen Antigen-1-negativen Tumoren benutzt werden.

Die aufgrund ihrer Reaktivität mit Antigen 2 charakterisierten monoklonalen Antikörper können zur diagnostischen Erkennung und Unterscheidung der > 200 KD-Antigen tragenden Gewebe (Endothelien, Gefäßtumore) von anderen Antigen-2-negativen Gewebe benutzt werden.

Weiterhin können alle die das oben beschriebene Antigen erkennenden Antikörper dazu benutzt werden, das von ihnen erkannten Antigen in Körperflüssigkeiten nachzuweisen oder in radioaktiv markierter Form an die das Antige tragenden Gewebe in vivo zu binden und die Antigene nachzuweisen (Immunszintigraphie). Zusätzlich können diese monoklonalen Antikörper als Wirkstoffträger eingesetzt und zur Therapie maligner Erkrankungen benutzt werden. Eine weitere Anwendungsmöglichkeit stellt die Hemmung der Tumorzellmetastasierung nach in vivo-Applikation und Bindung der monoklonalen Antikörper an antigentragende metastasierende Tumorzellen dar. Zusätzlich können diese monoklonalen Antikörper ohne die Anwesenheit anderer Toxine für die antigentragenden Tumorzellen toxisch sein oder nach Bindung an die Tumorzellmembran zu Differenzierungsvorgängen führen, die bösartige Tumorzellen zu benignen Zellen werden lassen.

Für diagnostische Zwecke können die monoklonalen Antikörper je nach Test in einer Konzentration von 0,001 µg/ml bis 100 µg/ml eingesetzt werden.

Therapeutische Effekte werden in Mengen von 200 µg-100 mg/kg Körpergewicht erreicht.

## Patentansprüche

1. Monoklonale Antikörper, die ein Epitop, das auf der Zellmembran der OAT-75 kleinzelligen Lungenkarzinomzellinie, auf von der OAT-75 abgeleiteten und auf der nackten Maus transplantierten Tumoren, in Gefäßen, im Kapillarschlingenendothel der Glomeruli, in Darmbeinkammvenenendothelzellen und das auf einem Glykoprotein-Antigen (AG2) mit einem Molekulargewicht > 200 kD unter nicht reduzierenden Bedingungen, das serologisch nicht mit FVIIIR:AG identisch ist, nachweisbar ist, erkennen und die durch Immunisieren von Säugetieren mit Zellen der Zellinie OAT-75 hergestellt werden.

2. Epitop, das auf der Zellmembran der OAT-75 kleinzelligen Lungenkarzinomzellinie, auf von der OAT-75 abgeleiteten und auf der nackten Maus transplantierten Tumoren, in Gefäßen, im Kapillarschlingenendothel der Glomeruli, in Darmbeinkammvenenendothelzellen und das auf einem Glykoprotein-Antigen (AG2) mit einem Molekulargewicht > 200 kD unter nicht reduzierenden Bedingungen, das serologisch nicht mit FVIIIR:AG identisch ist, nachweisbar ist, dadurch gekennzeichnet, daß es von monoklonalen Antikörpern nach Anspruch 1 erkannt wird.

3. Verfahren zur Herstellung von monoklonalen Antikörper nach Anspruch 1, dadurch gekennzeichnet, daß Säugetiere mit Zellen der OAT-75-Zellinie immunisiert, Milzzellen aus einem solchen immunisierten Tier entnommen, mit der Zellinie X63-Ag8653 fusioniert, die Hybridome selektioniert und die monoklonalen Antikörper gewonnen werden.

4. Verfahren nach Anspruch 3, dadurch gekennzeichnet, daß die Säugetiere Mäuse sind.

5. Verwendung von monoklonalen Antikörpern nach Anspruch 1 in einem *in vitro* Diagnostikum.

6. Verwendung von monoklonalen Antikörpern nach Anspruch 1 als Träger für einen pharmazeutischen Wirkstoff.

7. Verwendung eines monoklonalen Antikörpers nach Anspruch 1 als Therapeutikum.

## Claims

1. A monoclonal antibody which recognizes an epitope which is detectable on the cell membrane of the OAT-75 small-cell lung carcinome cell line, on the tumors derived from OAT-75 and transplanted onto the nude mouse, in vessels, in the capillary loop endothelium of the glomeruli, in endothelial cells of iliac crest veins and on a glycoprotein antigen (AG2) which has a molecular weight > 200 kD under non-reducing conditions and which is not identical to FVIIIR:AG by serology, which antibody is prepared by immunizing mammals with cells of the OAT-75 cell line.

2. An epitope which is detectable on the cell membrane of the OAT-75 small-cell lung carcinome cell line, on the tumors derived from OAT-75 and transplanted onto the nude mouse, in vessels, in the capillary loop endothelium of the glomeruli, in endothelial cells of iliac crest veins and on a glycoprotein antigen (AG2) which has a molecular weight > 200 kD under non-reducing conditions and which is not identical to FVIIIR:AG by serology, which epitope is recognized by a monoclonal antibody as claimed in claim 1.

3. A process for the preparation of a monoclonal antibody as claimed in claim 1, which comprises the immunization of mammals with cells of the OAT-75 cell line, the removal of spleen cells from an animal immunized in this way and fusion of them with the cell line X63-Ag8653, the selection of the hybridomas and the obtaining of the monoclonal antibody.

4. The process as claimed in claim 3, wherein the mammals are mice.

5. The use of a monoclonal antibody as claimed in claim 1 in an in vitro diagnostic aid.

6. The use of a monoclonal antibody as claimed in claim 1 as a carrier for a pharmaceutical active compound.

7. The use of a monoclonal antibody as claimed in claim 1 as a therapeutic agent.

## Revendications

1. Anticorps monoclonaux qui reconnaissent un épitope qu'on peut déceler sur la membrane cellulaire de la lignée cellulaire OAT-75 du carcinome pulmonaire à petites cellules, sur des tumeurs dérivées de l'OAT-75 et transplantées sur la souris nude, dans des tissus, dans l'endothélium du réseau capillaire des glomérules, dans les cellules endothéliales de la veine iliaque, et qu'on peut déceler sur un antigène de glycoprotéine (AG2) ayant une masse moléculaire > 200 kD dans des conditions non réductrices, qui n'est pas identique à FVIIIR:AG du point de vue sérologique, lesquels anticorps sont préparés par immunisation de mammifères avec des cellules de la lignée cellulaire OAT-75.

2. Epitope qu'on peut déceler sur la membrane cellulaire de la lignée cellulaire OAT-75 du carcinome pulmonaire à petites cellules, sur des tumeurs dérivées de l'OAT-75 et transplantées sur la souris nude, dans des tissus, dans l'endothélium du réseau capillaire des glomérules, dans les cellules endothéliales de la veine iliaque, et qu'on peut déceler sur un antigène de glycoprotéine (AG2) ayant une masse moléculaire > 200 kD dans des conditions non réductrices, qui n'est pas identique à FVIIIR:AG du point de vue sérologique, caractérisé en ce qu'il est reconnu par des anticorps monoclonaux selon la revendication 1.

3. Procédé pour préparer des anticorps monoclonaux selon la revendication 1, caractérisé en ce qu'on immunise des mammifères avec des cellules de la lignée cellulaire OAT-75, on prélève des cellules de la rate d'un de ces animaux immunisés, on fusionne lesdites cellules avec la lignée cellulaire X63-Ag8653, on sélectionne les hybridomes et on récupère les anticorps monoclonaux.

4. Procédé selon la revendication 3, caractérisé en ce que les mammifères sont des souris.

5. Utilisation d'anticorps monoclonaux selon la revendication 1 dans un agent de diagnostic in vitro.

6. Utilisation d'anticorps monoclonaux selon la revendication 1 comme véhicule pour un principe actif pharmaceutique.

7. Utilisation d'un anticorps monoclonal selon la revendication 1 comme agent thérapeutique.
